# EUROPEAN PATENT APPLICATION

(11) **EP 3 051 843 A1**
(43) Date of publication of application: **03.08.2016**
(21) Application number: 15153286.8
(22) Date of filing: 30.01.2015
(51) Int. Cl.: H04R 25/00, A61F 11/08, B29C 67/00, H04R 1/10

(54) **Method for manufacturing a custom fit ear plug element**

(71) Applicant: LUXeXcel Holding B.V., 4416 PX Kruiningen (NL)
(72) Inventor: Van de Vrie, Richard, NL-4471 NC Wolphaartsdijk (NL)
(74) Representative: Loock, Jan Pieter

(57) **Abstract**

The present invention suggest a method for manufacturing a custom fit ear plug element, wherein in a first step an anthropometric measurement of an auditory canal of a customer is performed, wherein in a third step the ear plug element is printed based on the result of the anthropometric measurement of the auditor canal, wherein the ear plug is printed by using a silicone-based printing ink

## Description

### Background

The present invention relates to a method for manufacturing a custom fit ear plug element.

Ear plug elements such as hearing aids, hearing protection or headphones are well known in the state of the art. Typically the ear plug elements are inserted into an auditory canal of the user and stay there over a longer period. Thus it is desirable to provide an ear plug element that has a high wearing comfort and good acoustic insulation. A way for improving the wearing comfort is individualizing the form of the ear plug element corresponding to the auditory canal. Such custom fit ear plug element requires adequate measuring methods that scan the auditory canal properly. An example for such a measuring method is disclosed in EP 1 314 000 B1.

However the quality of the individualized ear plug element is limited by the cooperation of measuring the ear plug element properly on the one hand and manufacturing the ear plug element in dependency on the results of the anthropometric measurement on the other hand.

### Summary

It is an object of the present invention to provide an ear plug element having a comparatively high wearing comfort and superior acoustic insulation.

The object is solved by a method for manufacturing a custom fit ear plug element for a customer, wherein in a first step an anthropometric measurement of an auditory canal of the customer is performed, wherein in a second step a customized shape for the ear plug element is determined in dependency on the anthropometric measurement, wherein in a third step the ear plug element corresponding to the customized shape is printed, wherein the ear plug element is printed by using a silicone-based printing ink, in particular inkjet printed by using the silicone-based printing ink.

Compared to the state of the art the method according to the present invention has the advantage of measuring the auditory canal and subsequently printing a custom fit ear plug element comprising a silicone-based printing ink. As a result of using a silicone-based printing ink a comparatively smooth and accurate ear-plug element is provided that is individually adapted to the auditory canal of the customer.

Preferably the ear plug element is part of a hearing aid, a headphone or an ear plug that is intended for sound insulation. In particular, it is provided that the ear-plug element is additionally adapted to its function, such as sound insulation and/or sound improvement. Preferably anthropometric measurement means the measurement of ear parameters such as an inner ear diameter, the topography of the auditory canal and/or the depth of the auditory canal accessible for the ear plug element, wherein these individual ear parameters differs from customer to customer. In particular, the auditory canal comprises the region in which the ear plug element is inserted during its use.

According to a preferred embodiment of the present invention it is provided that the anthropometric measurement is performed by using a formable sample, in particular a wax-type sample, and/or by using an inside ear scanning device, in particular a 3D-scanning device. Preferably it is provided that the anthropometric measurement includes a topographic mapping of the inside of the auditory canal. In particular, it is provided that the inside ear scanning device scans the inside area of the auditory canal and/or its topography. Furthermore it is thinkable that the wax-type sample is a replica of the inside of the auditory canal of the customer. In particular, the wax-type sample is a preform of at least a part of the ear plug element, wherein the flexible wax-type sample is inserted in the auditory canal of the customer and is forced to adopt the shape of the inside area of the auditory canal. It is also considerable that the anthropometric measurement is based on a plurality of pictures that are collected and subsequently evaluated in order to get a three dimensional impression of the inside of the auditory canal.

According to a preferred embodiment of the present invention it is provided that in the third step droplets having a volume of less than 10 Picoliters, preferably less than 5 Picoliters or more preferably less than 2 Picoliters are deposited for printing the ear plug element. It is herewith advantageously possible to generate an ear plug element having a comparatively smooth surface whilst maintaining some flexibility, thus without the drawbacks of the rigidity of acrylic based 3D printed ear plugs. Thus it is possible to improve the wearing comfort of the ear plug element dramatically. In particular it is provided that the ear plug element is printed by a droplet-on-demand process, wherein droplets are deposited next and/or above each other in order to generate the three dimensional structure of the ear plug element. According to a preferred embodiment of the present invention it is provided that the deposited droplets are cured, in particular by using light, such as for example UV- and/or IR- light. In particular, it is provided that the droplets are deposited and at least a part of a layer comprising these droplets is cured, wherein subsequently other droplets forming another layer are deposited onto the cured layer. Thus successively the ear plug element is realized layer by layer. Preferably the printing process of the final layers, i. e. those layers that form the surface of the ear plug element, is adapted for smoothing the surface. For example the curing process is paused till the deposited droplets spread enough for forming a smooth surface, wherein a viscosity of the printing ink is preferably adapted for such a spread process. Thus the probability for an unwanted unevenness is advantageously reduced.

According to a preferred embodiment of the present invention it is provided that in the first step an outer ear of the customer is measured at least partially and in the second step the customized shape of the ear plug element is determined in dependency on the measurement of the outer ear. The outer ear comprises for example an auricle, an ear cartilage and/or an earlobe. By considering other aspects of the anatomy of the ear for determining the shape of the customized ear plug element it is advantageously possible to further improve the wearing comfort.

According to a preferred embodiment of the present invention it is provided that in the first step a dataset comprising information of the anthropometric measurement is realized, wherein the dataset is transferred to a printing device for printing the ear plug element. The use of the dataset allows an effective coupling between the measurement process and the printing process. It is herewith thinkable that the dataset is transferred via an online connection or by using a hardware component such as an USB-stick. In particular, the dataset is saved or transferred as CAD -file or ASCII-file.

According to a preferred embodiment of the present invention it is provided that the droplets are deposited onto a non-individualized basic skeleton. Such a non-individualized basic skeleton is preferably made of plastic. By using the non-individualized basic skeleton as starting point for the printing process the manufacturing process is advantageously accelerated. It is also thinkable that the printed ear plug element is a casing for the basic skeleton, wherein the ear plug element is attached to the basic skeleton after the printing process has been finished. In particular, the skeleton is printed in a preparation step, wherein the skeleton is preferably printed by a three dimension printing process using a non-silicon based printing ink. Furthermore it is thinkable that the basic skeleton comprises electrical components that for example need to be integrated into a hearing aid or a headphone.

According to a preferred embodiment of the present invention it is provided that in the third step the droplets are deposited by an inkjet printer. In particular, the inkjet printer comprises a nozzle for ejecting droplets. It is herewith advantageously possible to deposit droplets fast and accurately. As a consequence the use of the inkjet printer guarantees a fast way of producing the custom fit ear plug element having a comparatively high quality.

According to a preferred embodiment of the present invention it is provided that in the third step a first part of the ear plug element having a first smoothness and a second part of the ear plug element having a second smoothness are printed. Herewith it is thinkable that the first part and the second part are printed subsequently or simultaneously. In particular it is provided that different regions of the printed ear plug element have different smoothness, i. e. the first smoothness differs from the second smoothness. For realizing different smoothness it is considerable to deposit in the area of the first part of the ear plug element a first kind of silicon based printing ink and in the area of the second part of the ear plug element a second kind of silicon based printing ink and/or a non-silicon based printing ink. Furthermore it is thinkable that the ear plug element is printed such that the mass or density of the printed ear plug element varies across the ear plug element. Thus the wearing comfort is further improved by the mass distribution across the ear plug element.

According to the preferred embodiment of the invention it is provided that in the second step
-- a position and/or a structure of the first part of the ear plug element and/or
-- a position and/or a structure of the second part of the ear plug element are determined in dependency on the anthropometric measurement. Thus it is advantageously possible to adapt the smoothness such that the wearing comfort is further improved.

According to a preferred embodiment of the present invention it is provided that in the first step the complexion of a customer's skin is identified. In particular the customer's skin is identified in the region of the ear of the customer.

According to a preferred embodiment of the present invention it is provide that a printing ink color is adapted to the complexion of the customer's skin. In order to realize hearing aids or head phones which are difficult to recognize or see it is herewith possible to adapt the color of the printing material. Thus an additional coloring of the hearing aid or head phone and therefore another manufacturing step can be avoided. In particular, it is provided that the silicone based printing ink is mixed with a dyestuff or a pigment for adjusting the color of the finished ear plug element.

According to a preferred embodiment of the present invention it is provided that a custom fit ear plug element for a hearing aid or a headphone is printed.

Another aspect of the present invention is a system for manufacturing an ear plug element by a method according to the present invention, wherein the system comprises a measurement device for an anthropometric measurement of the auditory canal and a printing device for printing an ear plug element.

It is herewith advantageously possible to manufacturing custom fit ear plug elements having a comparatively high wearing comfort by a printing process for three dimensional structures, preferably by a droplet-on-demand printing process. It is herewith considerable that the system is integrated in a kind of vending machine for producing the ear plug element, or the measuring device and the printing device communicates with each other, for example via a LAN- or WLAN- connection or via Bluetooth.

Another aspect of the present invention is an ear plug element printed by a method according to the present invention.

These and other characteristics, features and advantages of the present invention will become apparent from the following detailed description, taken in conjunction with the accompanying drawings, which illustrate, by way of example, the principles of the invention. The description is given for the sake of example only, without limiting the scope of the invention. The reference figures quoted below refer to the attached drawings.

### Brief description of the drawings

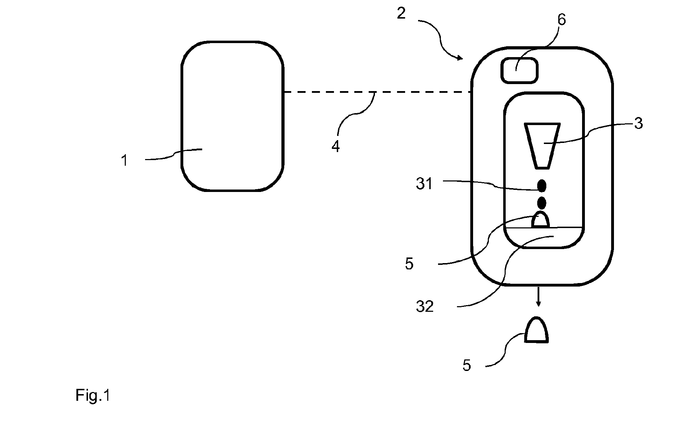

- **Figure 1**: shows schematically a method for manufacturing an custom fit ear plug element according to an exemplary embodiment of the present invention.

### Detailed description

The present invention will be descripted with respect to particular embodiments and with the reference to certain drawings but the invention is not limited thereto but only by the claims. The drawings described are only schematic and are non-limiting. In the drawings, the size of some elements may be exaggerated and not drawn on scale for illustrative purposes.

Where an indefinite or definite article is used when referring to a singular noun, e. G. "a","an", "the", this includes a plurals of the noun unless something else is specifically stated.

Furthermore, the terms first, second, third and the like in the description and in the claims are used to distinguishing between similar elements and not necessarily for describing a sequential or chronological order. It is to be understood that the terms so used are interchangeable under appropriate circumstances and that the embodiments of the invention described herein are capable of operation in other sequences than described of illustrated herein.

In **figure 1** schematically a method for manufacturing an ear plug element 5 according to an exemplary embodiment of the present invention is illustrated. In particular, it is herewith provided to manufacture at least a part of a hearing aid by a printing process, in particular by a droplets-on-demand printing process. For improving the wearing comfort of said ear plug element it is further provided that an individualized ear plug element 5 is printed based on ear parameter that are evolved by a scanning process. Preferably a measurement device 1 is provided, wherein the measurement device 1 scans the inside of an auditory canal of a customer in order to get information about the topography of the auditory canal. Based on these evolved data an ear plug element 5 is printed. In particular the shape of the ear plug element 5, in particular its outer surface, is adapted to the topography of the auditory canal, i. e. the inner surface of the auditory canal. Preferably the ear plug element 5 is designed complementary to the topography of at least a region inside the auditory canal. Furthermore it is provided that the result of the anthropometric measurement is transferred to the printing device 2 via a communication channel 4, using for example a LAN- or WLAN- connection. Preferably the printing device 1 and/or the measurement device 2 comprises a control device 6 that determines printing information based on the ear parameters that result from the anthropometric measurement of the customer's auditory canal. Preferably the printing process is started as soon the ear parameters have been transformed to the printing information. In particular, it is provided that in the third step droplets 31 of a silicone based printing ink are deposited onto a substrate 32 and subsequently the deposited droplets are cured by light, preferably UV-light or IR-light. Preferably the silicone-based printing ink comprises an UV curable liquid monomer which becomes a polymer by curing. It is further conceivable that the printing material has different colors and is individual adapted to the preferences of the customer. Furthermore it is provided to use a printing head for depositing the droplets of printing material. Furthermore the silicon printing material has a viscosity. Consequently the droplets 31 of the material may spread or diffuse. In particular it is provided, that a plurality of droplets 31 of printing material is deposited onto a substrate 32 and subsequently cured using light in particular UV-light. As a result the cured droplets 31 generate a part of the printed ear plug element 5. Preferably it is provided that the process of depositing the droplets 31 onto the substrate 32 and/or the printed part of the ear plug element 5 and subsequently curing the droplets 31 is repeated iteratively till the ear plug element 5 is finished. For depositing the droplets 31 on the printed part of the ear plug element 5 and/or the substrate 32 preferably a nozzle 3 is used. The nozzle 3 ejects printing material in shape of the silicone based droplets 31 toward the substrate 32 and/or the printed part of the ear plug element 5. In particular, the silicon based printing material may be ejected by a print head of an inkjet printer, wherein the print head is moveable and distributes the droplets 31 of the printing material such that a layer including a plurality of droplets 31 is formed. A layer corresponds to an arrangement of droplets 31 within a plane that is more or less parallel to the substrate 32 and/or the printed part of the ear plug element 5, for instance. In particular, it is also considerable that the nozzle 5 or the head of the inkjet printer moves and consequently several droplets 31 are arranged next to and/or above each other forming the layer.

### Reference signs:

- 1: measurement device
- 2: printing device
- 3: nozzle
- 4: communication canal
- 5: ear plug element
- 6: control unit
- 31: droplet
- 32: substrate

## Claims

1. Method for manufacturing a custom fit ear plug element (5) for a customer, wherein in a first step an anthropometric measurement of an auditory canal of the customer is performed, wherein in a second step a customized shape for the ear plug element is determined in dependency on the anthropometric measurement, wherein in a third step the ear plug element (5) corresponding to the customized shape is printed, wherein the ear plug element (5) is printed by using a silicone-based printing ink.

2. Method according to claim 1, wherein the anthropometric measurement is performed by using a formable sample, in particular a wax-type sample, and/or by using an inside ear scanning device, in particular a 3D-scanning device.

3. Method according to one of the preceding claims, wherein in the third step droplets (31) having a volume of less than 10 Picoliters, preferably less than 5 Picoliters or more preferably less than 2 Picoliters are deposited for printing the ear plug element (5).

4. Method according to one of the preceding claims, wherein the deposited droplets (31) are cured, in particular by using light.

5. Method according to one of the preceding claims, wherein in the first step an outer ear of the customer is measured at least partially and in the second step the customized shape of the ear plug element (5) is determined in dependency on the measurement of the outer ear.

6. Method according to one of the preceding claims, wherein in the first step a dataset comprising information of the anthropometric measurement is realized, wherein the dataset is transferred to a printing device for printing the ear plug element (5).

7. Method according to one of the preceding claims, wherein the droplets (31) are deposited on a non-individualized basic skeleton.

8. Method according to one of the preceding claims, wherein in the third step the droplets (31) are deposited by a printing device (2), preferably by an inkjet printer.

9. Method according to one of the preceding claims, wherein in the third step a first part of the ear plug element (5) having a first smoothness and a second part of the ear plug element (5) having a second smoothness are printed.

10. Method according to claim 9, wherein in the second step
-- a position and/or a structure of the first part of the ear plug (5) element and/or
-- a position and/or a structure of the second part of the ear plug element (5) are determined in dependency on the anthropometric measurement.

11. Method according to one of the preceding claims, wherein in the first step the complexion of a customer's skin is identified.

12. Method according to claim 11, wherein in the third step the printing ink color is adapted to the identified complexion of the customer's skin.

13. Method according to one of the preceding claims, wherein a custom fit ear plug element (5) for a hearing aid or a headphone is printed.

14. System for manufacturing an ear plug element by a method according to one of the preceding claims, wherein the system comprises a measurement device (1) for an anthropometric measurement of the auditory canal and a printing device (2) for printing an ear plug element (5).

15. Ear plug element printed by a method according to one of the claims 1 to 13.
